Europäisches Patentamt

European Patent Office

Office européen des brevets

(19)

(11) Veröffentlichungsnummer : **0 111 257
B2**

(12) **NEUE EUROPÄISCHE PATENTSCHRIFT**

(45) Veröffentlichungstag der neuen Patentschrift :
04.05.88

(51) Int. Cl.⁴ : **C 07 C 45/50, C 07 C 47/02**

(21) Anmeldenummer : 83112049.8

(22) Anmeldetag : 01.12.83

(54) Verfahren zur Hydroformylierung von Olefinen.

(30) Priorität : 11.12.82 DE 3245883

(43) Veröffentlichungstag der Anmeldung :
20.06.84 Patentblatt 84/25

(45) Bekanntmachung des Hinweises auf die Patenterteilung : 17.04.85 Patentblatt 85/16

(45) Bekanntmachung des Hinweises auf die Entscheidung
über den Einspruch : 04.05.88 Patentblatt 88/18

(84) Benannte Vertragsstaaten :
AT DE FR GB NL

(56) Entgegenhaltungen :
EP-A- 0 016 285
EP-A- 0 062 282
AT-B-   360 969
DE-A- 3 102 281
DE-C-   935 126
GB-A- 1 387 657
J. Falbe "New Syntheses with Carbon Monoxide",
Springer Verlag Berlin, Heidelberg, New York, 1980,
S. 53-55, 96-98, 174-177, 49
J. Falbe "Synthesen mit Kohlenmonoxyd", Springer-
Verlag Berlin, Heidelberg, New York, 1967, S. 19

(73) Patentinhaber : Ruhrchemie Aktiengesellschaft
Bruchstrasse 219
D-4200 Oberhausen 13 (DE)

(72) Erfinder : Cornils, Boy, Dr. Dipl.-Chem.
Friedrich-Ebert-Strasse 45
D-4220 Dinslaken (DE)
Erfinder : Hibbel, Josef, Dipl.-Ing.
Bruchsteg 13
D-4200 Oberhausen 11 (DE)
Erfinder : Kessen, Gunther, Dr. Dipl.-Chem
Vennstrasse 6
D-4200 Oberhausen 11 (DE)
Erfinder : Konkol, Werner, Dr. Dipl.-Chem.
Lützowstrasse 40 A
D-4200 Oberhausen 11 (DE)
Erfinder : Lieder, Bernhard
Siegfriedstrasse 61
D-4250 Bottrop (DE)
Erfinder : Wiebus, Ernst
Ferdinandstrasse 77
D-4200 Oberhausen 14 (DE)
Erfinder : Kalbfell, Heinz, Dipl.-Ing.
Eichenstrasse 20
D-4235 Schermbeck (DE)
Erfinder : Bach, Hanswilhelm, Dr. Dipl.-Chem.
Alleestrasse 56
D-4100 Duisburg 11 (DE)

(74) Vertreter : Reichelt, Karl-Heinz, Dr.
Ruhrchemie Aktiengesellschaft Abt. PLD Postfach 13
01 60
D-4200 Oberhausen 11 (DE)

**Beschreibung**

Die Erfindung betrifft eine besonders vorteilhafte Variante der Oxosynthese in zwei Stufen. Hierbei werden die Abgase aus Oxoanlagen, die unter niedrigen Reaktionsdrücken betrieben werden, und die noch erhebliche Anteile unumgesetztes Olefin enthalten, in geeigneter Weise gesammelt und anschließend in technischen Oxoanlagen unter höheren Drücken umgesetzt.

Für die Hydroformylierung von Olefinen sind neben vielen Laborverfahren drei großtechnische Prozesse bekannt geworden. Beim Hochdruck-Verfahren (HD-Verfahren) werden die Einsatzolefine je nach Zahl der Kohlenstoffatome des Olefins und je nach gewünschtem Reaktionsprodukt Aldehyde oder Alkohole, bei Drükken von 100 bis 350 bar und Temperaturen von 120 bis 180 °C mit Synthesegas umgesetzt Aktiver Katalysator der Reaktion ist Hydridokobalttetracarbonyl, das als solches eingesetzt oder während der Reaktion aus geeigneten Kobaltverbindungen gebildet werden kann. Diese HD-Verfahren sind unter entsprechenden Bedingungen im allgemeinen durch hohe Olefinumsätze gekennzeichnet, so daß auf aufwendige Maßnahmen zur Olefinrückgewinnung und -rückführung verzichtet werden kann. Das Abgas der HD-Reaktion enthält deshalb neben wenig Einsatzolefin vor allem das entsprechende Paraffin und Synthesegas.

Die Nachverarbeitung des Abgases kommerzieller Anlagen in einer weiteren Hochdruckstufe ist wegen des nahezu vollständigen Umsatzes wirtschaftlich schon deshalb nicht sinnvoll, da dann das unumgesetzte Propylen in geringer Konzentration enthaltende Abgas erneut komprimiert werden müßte.

Beim Mitteldruck-Vefahren (MD-Verfahren) werden Kobaltkatalysatoren, die mit Phosphinen modifiziert sind, verwendet. Der niedrige Reaktionsdruck von 50 bis 100 bar hat u. a. eine Senkung des Olefinumsatzes zur Folge. Die durch Phosphine modifizierten Kobaltkatalysatoren wirken unter den angewendeten Arbeitsbedingungen stark hydrierend ; daher ist das nicht umgesetzte Olefin aus der Reaktion im allgemeinen erheblich mit Paraffin der gleichen Kohlenstoffatomzahl verunreinigt. Weil der Wiedereinsatz des Olefins eine aufwendige Olefin/Paraffintrennung erfordert, wird auf eine Olefingewinnung und -rückführung und damit eine wirtschaftlich optimale Verfahrensführung meistens verzichtet.

Im Oxo-Niederdruck-Verfahren (ND-Verfahren) schließlich werden durch Phosphin modifizierte Rhodiumkatalysatoren eingesetzt, die eine Hydroformylierung bei Temperaturen von 60 bis 150° C und Drücken von 10 bis 80 bar ermöglichen. Der Vorteil des ND-Verfahrens besteht vor allem in der hohen Selektivität der Hydroformylierungsreaktion.

Der Nachteil aller ND-Verfahren ist, daß die tiefen Reaktionstemperaturen bei Einsatz technischer Propylen-Qualitäten nur einen begrenzten Olefinumsatz erlauben, so daß aus wirtschaftlichen Gründen das Einsatzolefin wiedergewonnen und zurückgeführt werden muß. Auf der anderen Seite haben die durch Liganden modifizierten Rhodiumkatalysatoren den Vorteil, daß ihre Hydrierneigung bedeutend geringer ist als diejenige der Kobalt-Phosphin-Katalysatoren. Daher ist die auf die Hydrierung des Einsatzolefins zurückzuführende Paraffinbildung geringer als bei MD-Verfahren. Aber auch bei der ND-Oxosynthese mit kontinuierlicher Wiedergewinnung und kontinuierlichem Wiedereinsatz des je Reaktordurchgang nicht umgesetzten Einsatzolefins muß eine Paraffin/Olefin-Trennung vorgesehen werden. Bei Verzicht auf eine derartige Trennung gehen mit dem für die Oxostufe wertlosen Paraffin unverhältnismäßig hohe Olefinanteile verloren.

Die weiterhin denkbare Umsetzung des restlichen Propylens in angeschlossene ND-Anlagen würde die Installation einer unwirtschaftlichen Kaskadenschaltung nach sich ziehen.

Nach einer in der DE-OS 31 02 281 beschriebenen Arbeitsweise führt man die Propylen enthaltenden Abgase bereits bestehender HD-Stufen in eine ND-Anlage ein und ergänzt bzw. ersetzt so herkömmliche HD-Oxoanlagen schrittweise durch ND-Verfahren.

Ein Nachteil dieses Verfahrens ist, daß das gesamte Olefin, also auch der letztlich nur in der Niederdruckstufe umzusetzende Anteil zunächst komprimiert werden muß. Darüber hinaus ist eine ND-Anlage zur Verwertung des restlichen Propylens aus HD-Anlagen sehr aufwendig.

Die GB-A1-1 387 657 betrifft die Hydroformylierung eines Olefins nach einem ND-Verfahren in einer 1. Reaktionszone und die Umsetzung des aus dieser 1. Reaktionszone herrührenden nicht umgesetzten Olefins in einer 2. Reaktionsstufe, nachdem Aldehyd und gegebenenfalls auch Alkohol zuvor abgetrennt worden sind. In beiden Reaktionsstufen werden gleichartige Katalysatoren eingesetzt, unter Anpassung der Reaktionsbedingungen im 2. Reaktor an die Zusammensetzung des Gasgemisches aus dem 1. Reaktor. Diese Anpassung kann auch eine begrenzte Druckerhöhung umfassen, sie bedeutet aber nicht, daß sich dem ND-Verfahren der 1. Stufe ein HD-Verfahren in der 2. Stufe anschließt.

Die vorstehend geschilderten Nachteile vermeidet ein Verfahren zur Hydroformylierung von Olefinen mit 2 bis 5 Kohlenstoffatomen nach einem Niederdruckverfahren mit Rhodium/organischen Phosphin-Katalysatoren. Hierbei wird das nicht umgesetztes Olefin enthaltende Abgas der Niederdruckanlage ohne weitere Zwischenbehandlung auf 100 bis 350 bar komprimiert und zur Hydroformylierung in einer Hochdruckanlage bei Temperaturen von 120 bis 180 °C unter Verwendung von Kobaltkatalysatoren eingesetzt.

Überraschenderweise steht der Durchführbarkeit der erfindungsgemäßen Arbeitsweise nicht entgegen, daß das Abgas der ND-Anlage (Niederdruckstufe) üblicherweise in Spuren Phosphine enthält, die in der Hochdruckstufe als Gift für den Kobaltkatalysator wirken. In diesem Zusammenhang wird auf die Angaben in J. Falbe, « New Synthesis with Carbon Monoxide », Springer Verlag 1980, Seite 53 verwiesen, die besagen, daß ligandmodifizierte Kobaltkatalysatoren reduzierte Hydroformylierungsaktivität aufweisen, jedoch ausgeprägte Hydrierungsaktivät besitzen. Die Folge ist, daß als Reaktionsprodukte nur Alkohole und keine Aldehyde erhalten werden und weiterhin 15 % der Olefine in Paraffine übergehen. Es hat sich herausgestellt, daß trotz dieses Phosphingehaltes das Abgas mit hohem Umsatz und ohne Schädigung des Katalysators in der HD-Anlage (Hochdruckstufe) des kombinierten Prozesses eingesetzt werden kann. Der Vollständigkeit der Reaktion steht auch nicht entgegen, daß das Abgas der ND-Anlage verhältnismäßig viel Wasserstoff enthält, der zu einer Absenkung der Aldehydausbeute in der Hochdruckstufe führen sollte. Schließlich ist es im Rahmen des erfindungsgemäßen Verfahrens nicht notwendig, in der Niederdruckstufe hochreines Olefin einzusetzen. Es reicht vielmehr aus, handelsübliche Olefine zu verwenden, die noch Bestandteile gesättigter Kohlenwasserstoffe enthalten, im Falle des Propylens z. B. etwa 5 % Propan. Die Paraffine können mit dem in der Hochdruckstufe anfallenden Abgas aus dem Prozeß ausgeschleust werden.

Die Durchführung der Umsetzungen in der Niederdruckstufe und in der Hochdruckstufe des Gesamtverfahrens erfolgt in bekannter Weise. In der Niederdruckstufe arbeitet man bei 10 bis 80 bar Druck und Temperaturen von 60 bis 150 °C. Als Katalyator findet Rhodium zusammen mit organischen Phosphinen Anwendung. Das Rhodium wird metallisch oder als Verbindung, z. B. als Rhodiumchlorid, Rhodiumoxid oder Rhodiumacetat in Konzentrationen von 200 ppm bis 800 ppm eingesetzt. Als Phosphine haben sich insbesondere aromatische Phosphine wie Triphenylphosphin bewährt. Sie können auch neben oder zusammen mit hochsiedenden Nebenprodukten der Synthese Bestandteil des Reaktionsmediums sein.

Das Atom- bzw. Molverhältnis von Rhodium zu Phosphin kann — je nach Reaktionsmedium — 1 : 2000 bis 1 : 30 betragen. Als Reaktionsmedium kann bei Einsatz wasserlöslicher Phosphine auch Wasser Anwendung finden. Wasserlöslich sind Phosphine, deren organische Reste carboxyliert oder sulfoniert worden sind.

Das den Niederdruckreaktor verlassende Abgas hat im allgemeinen eine Zusammensetzung in folgenden Bereichen : 26-40 Vol.-% Propylen, 6-20 Vol.-% Propan, 20-40 Vol.-% Wasserstoff und 20-40 Vol.-% Kohlenmonoxid. Es wird ohne weitere Zwischenbehandlung auf 100 bis 350 bar Druck komprimiert und bei Temperaturen von 120 bis 180 °C weiter umgesetzt. Als Katalysator findet Kobalt in metallischer Form oder als Verbindung Anwendung. Die Umsetzung läuft im Reaktionsprodukt als Reaktionsmedium ab. Das Reaktionsprodukt wird entspannt und von dem in ihm gelösten Kobalt befreit. Dabei fällt ein Abgas an, dessen Zusammensetzung bei den technisch gebräuchlichen Verfahren folgende Werte hat : 1-2 Vol.-% Propylen, 5-16 Vol.-% Propan, 25-40 Vol.-% Wasserstoff und 45-55 Vol.-% Kohlenmonoxid.

Beispiel 1

Umsetzung von Propylen
nach dem HD-Verfahren (Vergleich)

In einem Versuchs-Hochdruckreaktor wird handelsübliches Propylen mit einem Propangehalt von 5 % bei 150 °C und unter Druck von 260 bar mit Synthesegas (CO : $H_2$=1 : 1) umgesetzt. Der Durchsatz beträgt 1 l Propylen/1 Reaktorvolumen, die Co-Konzentration 0,5 Gew.-%, bezogen auf die Reaktorbeschickung. Bei einem Umsatz von 98 %, bezogen auf den Propyleneinsatz, wird ein Reaktionsprodukt folgender Zusammensetzung erhalten :

| | |
|---|---|
| $C_4$-Aldehyde | 84,2 % |
| $C_4$-Alkohole | 2,9 % |
| $C_4$-Methylester | 5,1 % |
| Dicköl | 7,8 % |
| n/i-Verhältnis | 75 : 25 |

Daraus ergibt sich unter Einrechnung einer Propanbildung von 1 %, daß je 100 kg Propylen (100 %) 115,0 kg n-$C_4$-Wertprodukte gebildet werden.

Beispiel 2

Umsetzung von Propylen
nach dem ND-Verfahren (Vergleich)

In einem Versuchsreaktor wird handelsübliches Propylen mit einem Propangehalt von 5 % bei 120 °C und unter einem Druck von 20 bar mit Synthesegas (CO : $H_2$=1 :1) umgesetzt. Der Durchsatz beträgt 0,2 l Propylen/l Reaktorvolumen, die Rhodiumkonzentration 400 ppm, bezogen auf die Reaktorbeschickung. Bei einem Umsatz von 87 %, bezogen auf den Propyleneinsatz, wird ein Reaktionsprodukt folgender Zusammensetzung erhalten :

| | |
|---|---|
| $C_4$-Aldehyde | 98,9 % |
| $C_4$-Alkohole | 0,5 % |
| $C_4$-Methylester | 0,1 % |
| Dicköl | 0,6 % |
| n/i-Verhältnis | 90 : 10 |

Daraus errechnet sich unter Berücksichtigung einer Propanbildung von 0,2 % eine n-$C_4$-Wertproduktausbringung von 133,0 kg/100 kg Propylen (100 %).

Die zur Ausschleusung des Propans erforderliche Abgasmenge beträgt 29,4 Nm³/100 kg Propylen mit einem Propylengehalt von 23,4 % und einem Propangehalt von 9,2 %, der Rest ist $H_2$ und CO im Volumverhältnis von etwa 1 : 1.

Beispiel 3

## Umsetzung von Abgas des ND-Verfahrens nach dem HD-Verfahren

Propylenhaltiges Abgas des ND-Verfahrens wird komprimiert und unter den im Beispiel 1 angegebenen Bedingungen nach dem HD-Verfahren hydroformyliert. Bei einem Umsatz von 96,7 % bezogen auf eingebrachtes Propylen, verläuft die Hydroformylierung sogar mit einer geringfügig besseren Selektivität als bei Frischpropylen, was auf einen Verdünnungseffekt durch das Propan zurückzuführen sein dürfte.

Entsprechend der Zusammensetzung des Reaktionsproduktes

| | |
|---|---|
| $C_4$-Adehyde | 85,6 % |
| $C_4$-Alkohole | 2,9 % |
| $C_4$-Methylester | 4,5 % |
| Dicköl | 7,0 % |
| n/i-Verhältnis | 76 : 24 |

ergibt sich unter Berücksichtigung einer Propanbildung von 1 % eine Ausbeute an n-$C_4$-Wertprodukten von 115,2 kg/100 kg Propyleinsatz.

Unter Zugrundelegung eines Hydroformylierungseinsatzes von 29,4 $Nm^3$ Abgas der ND-Fahrweise mit einem Propylengehalt von 23,4 Vol.-% ≙ 12,8 kg, errechnet sich aus den Versuchshydroformylierungen entsprechend Beispiel 2 und 3 eine Gesamt-n-$C_4$-Wertproduktionsausbeute von 147,7 kg/100 kg Propylen. Diese Ausbeute liegt damit um 14,7 kg/100 kg Olefin über dem Wert der ND-Hydroformylierung ohne nachgeschaltete Umsetzung des propylenhaltigen Abgases in einem HD-Reaktor.

**Patentanspruch**

Verfahren zur Hydroformylierung von Olefinen mit 2 bis 5 Kohlenstoffatomen nach einem Niederdruckverfahren mit Rhodium/organischen Phosphin-Katalysatoren, dadurch gekennzeichnet, daß das nicht umgesetztes Olefin enthaltende Abgas der Niederdruckanlage ohne weitere Zwischenbehandlung auf 100 bis 350 bar komprimiert und zur Hydroformylierung in einer Hochdruckanlage bei Temperaturen von 120 bis 180 °C unter Verwendung von Kobaltkatalysatoren eingesetzt wird.

**Claim**

A process for the hydroformylation of olefins with 2 to 5 carbon atoms with rhodium/organic phosphine catalysts according to the low pressure process characterised in that the waste gas from the low pressure plant containing unreacted olefin is compressed to 100 to 350 bar without further intermediate treatment and used for hydroformylation in a high pressure plant at temperatures of 120 to 180 °C using cobalt catalysts.

**Revendication**

Procédé pour l'hydroformylation d'oléfines en $C_2$-$C_5$ selon un procédé basse pression avec des catalyseurs au rhodium/phosphine organique, caractérisé en ce que le gaz résiduaire de l'installation basse pression contenant de l'oléfine non transformée est comprimé à une pression de 100 à 350 bars sans un autre traitement intermédiaire et utilisé pour l'hydroformylation dans une installation haute pression à des températures de 120 à 180 °C utilisant des catalyseurs au cobalt.